# EUROPEAN PATENT APPLICATION

(11) **EP 3 904 529 A1**
(43) Date of publication of application: **03.11.2021**
(21) Application number: 19903766.4
(22) Date of filing: 27.12.2019
(51) Int. Cl.: C12Q 1/6806, C12M 1/00, C12M 1/34

(54) **METHOD AND PRODUCT FOR DETECTING EMBRYO HEALTH CONDITION BY USING BLASTOCYST CULTURE SOLUTION**

(30) Priority: 29.12.2018 CN 201811647225
(71) Applicant: Xukang Medical Science & Technology (Suzhou) Co., Ltd., Suzhou, Jiangsu 215123 (CN)
(72) Inventor: YAO, Yaxin, Suzhou, Jiangsu 215123 (CN); LI, Wenlu, Suzhou, Jiangsu 215123 (CN); MA, Jieliang, Suzhou, Jiangsu 215123 (CN); LU, Sijia, Suzhou, Jiangsu 215123 (CN)
(74) Representative: Latscha Schöllhorn Partner AG
(86) International application number: PCT/CN2019/129196
(87) International publication number: WO 2020/135713

(57) **Abstract**

The invention provides a method and a product for detecting the health status of an embryo by using a blastocyst culture medium. Specifically, the present invention provides an *in vitro* method for detecting the health status of embryos using blastocyst culture medium, characterized in that it comprises the steps of: (a) providing a first blastocyst culture system containing a blastocyst of *in vitro* culture on Day 5 (D5) to Day 6 (D6); (b) transferring the blastocysts into a second blastocyst culture system containing a fresh blastocyst culture medium, and culturing in the exchanged medium for a time of T1, thereby obtaining a spent blastocyst culture medium; (c) collecting the spent blastocyst culture medium, thereby obtaining a cell-free spent blastocyst culture medium, i.e. a test sample; and (d) performing a genetic assay to identify the health status of the blastocyst. The method can reduce the risk of being Interfered by contaminants from extra sperms and maternal-derived granulosa cells during IVF and ICSI treatment, and can accurately identify the health status of the embryo.

## Description

### Technical field

The invention relates to the fields of biomedicine and molecular cell biology, in particular to a method and product for detecting embryo health status by using a blastocyst culture medium.

### Background

Preimplantation Genetic Screen (PGS) detects the chromosomal status of embryos cultured in vitro to screen embryos with normal chromosomes to be implanted in the mother's uterus, thereby increasing the success pregnancy rate to about 60%. The biological samples required for various tests are one to several cells collected from embryos cultured in vitro, commonly known as biopsy, and the detection of these small numbers of cells reflects whether the chromosomes of the entire embryo are normal. Theoretically, the chromosome status of the aspirated cells is the same as that of other cells in the embryo. By testing these cells, it can be determined that whether the embryo's chromosome status is normal. It is generally believed that the biopsy of a few trophoblast cells at this time will not have an adverse effect on embryonic development. At present, judging from the health of the newborn baby, this operation has not been shown to have any health effects. However, the embryo manipulation for the cell sampling has a relatively high technology requirement. If the operation is improper, it will cause serious damage to the embryo. If the damage is too severe, the embryo development can be terminated; and even if the cell sampling is performed well, it will inevitably cause cell loss and slight damage to embryo. In addition, the technology has only appeared for a short time (only a few years), and although there has been no evidence that cell loss and minor damage would have adverse effects on embryonic development and postnatal health, it remains to be seen that whether it has a long-term impact on human life-long health. Moreover, in some cases, the chromosomal status of a few cells obtained by sampling appears different from the chromosomal status of other cells in the embryo, resulting in false test results.

Recently, a non-biopsy technique has been investigated that does not take embryonic cells and only uses discarded blastocyst culture medium to reflect the health of embryos. The discarded blastocyst culture medium is processed and tested and analyzed. The test analysis results indirectly reflect the health status of blastocyst embryos with high accuracy. The technique does not take any components of the embryo itself, does not have ethical issues. The technique does not cause any loss to the embryos, and eliminates the hidden danger to health effects. In addition, the technique is simple and safe to operate, and can reflect the status of embryo with a relatively high accuracy.

The conventional process to carry out the technique includes: fresh in vitro fertilized oocytes are cultured in a culture medium to Day 3 (D3), and then the embryos are transferred to a blastocyst culture medium on Day 3 (D3) and cultured to Day 5 (D5) or Day6 (D6), and the blastocysts are removed on D5 or D6 for embryo grading. The freezing operation is carried out immediately if the embryos reach the grade for freezing. The remaining blastocyst culture medium is the test sample that needs to be collected. Clinically, an in vitro fertilization is usually performed by one of the processes, IVF and ICSI. In the process of IVF, an oocyte is fertilized by co-incubation with more than one sperm; and in the process of ICSI, an oocyte is fertilized by injection of a single sperm. In IVF, there are usually extra sperm hanging around the zona pellucida of embryos. In order to avoid the interference of the sperm's paternal DNA, the ICSI fertilization is generally used.

Galluzzi L, et al. (2015, Extracellular embryo genomic DNA and its potential for genotyping applications, Future Science OA 1(4): FSO62) describes a method, in which the culture medium is exchanged on Day 3 (D3) to a blastocyst culture medium, and an embryo is cultured in the blastocyst culture medium from Day 3 (D3) to Day 5 or 6 (D5/D6), and then the spent blastocyst culture medium is collected as a test sample. Vera-Rodriguez M. et al. (Origin and composition of cell-free DNA in spent medium from human embryo culture during preimplantation development, Hum Reprod. 2018 Apr 1;33(4):745-756) describes a method only for the ICSI fertilization, in which a medium exchange is also made on D3 to transfer embryos to a blastocyst culture medium and culture from D3 to D5/D6, and then the spent blastocyst culture medium is collected as a test sample. CN201510746098.X reports a method which is also used for only ICSI fertilization, and the method includes culturing embryos to D5/D6 after culture medium exchange on D3. Compared with the trophectoderm biopsy detection method, those conventional processes for non- non-biopsy detection have greatly improved the accuracy of embryo detection results, but still has a false-negative rate of about 15%, mainly because the test sample is inevitably interfered by external substances, especially the interfering substances of maternal origin.

In some literatures, methods that are slightly different from those conventional processes are described. For example, Medium-Based Noninvasive Preimplantation Genetic Diagnosis for Human -Thalassemias-SEA, Medicine (Baltimore). 2015 Mar;94(12):e669, reports a method, which includes a first medium exchange on D3, and a second medium exchange on D4, followed by a culture from D4 to D5/D6, and a collection of the spent blastocyst culture medium as a test sample. Kuznyetsov V. (Evaluation of a novel non-invasive preimplantation genetic screening approach, PLoS One. 2018 May 10;13(5):e0197262) reports a sampling method only for ICSI but not for IVF, in which fresh embryos are cultured to D5 with a medium exchange on D4, and the blastocoel fluid is collected after shrinkage of blastocoeles using a laser pulse. In those documents, the in vitro incubation time for sampling is shifted to Day 4 (D4) and samples are collected on D5/D6. On the one hand, the 12-36hr in vitro incubation time of the test sample is still long, so that it is still unavoidable to be interfered by external substances, thereby decreasing the ratio of embryo-derived DNAs, and the accuracy of embryo detection results has not been significantly improved, and there is still a false-negative rate of about 15%, and it is impossible to apply to both ICSI sampling and IVF sampling. On the other hand, embryos are evaluated or operated on D4, and the additional step may have an unpredictable effect on embryo development. Day 4 (D4) embryos usually develop into morula-stage embryos, which is a key period for embryos to develop into blastocysts. Morula-stage is followed immediately by blastocyst stage. The blastocyst formation rate is a key indicator of embryo development. After a blastocyst forms, *in vitro* manipulations such as transplantation or freezing can be considered. Therefore, *in vitro* operations such as changing the culture medium of embryos at D4 morula-stage are usually not performed clinically, so as not to affect the blastocyst formation rate.

However, in current detection methods, the test samples are inevitably interfered by external substances, especially maternal substances, resulting in a high false-negative rate and cannot apply to both ICSI sampling and IVF sampling.

Therefore, there is an urgent need in the art to develop a method and product suitable for both ICSI and IVF that can improve the accuracy and reduce the false-negative rate by using blastocyst culture medium as a test sample to detect the health of the embryo.

### Summary of the invention

The purpose of the present invention is to provide a method and product suitable for ICSI and IVF that can improve the accuracy and reduce the false-negative rate by using a blastocyst culture medium as a test sample to detect the health of the embryo.

The first aspect of the present invention provides an *in vitro* method for detecting the health status of a blastocyst by using a blastocyst culture medium, which includes the steps:
(a) providing a first blastocyst culture system, which comprises a blastocyst on Day 5 (D5) to Day 6 (D6) of *in vitro* culture;
(b) transferring the blastocysts into a second blastocyst culture system containing a fresh blastocyst culture medium, and culturing in the exchanged medium for a time of T1, thereby obtaining a spent blastocyst culture medium;
(c) collecting the spent blastocyst culture medium, thereby obtaining a cell-free spent blastocyst culture medium, i.e., a test sample; and
(d) performing a genetic assay to identify the health status of the blastocyst.

In a preferred embodiment, the first blastocyst culture system includes a system for de novo blastocyst culture (that is, the culture starts from D1 and does not undergo the change of the medium).

In another preferred embodiment, the first blastocyst culture system includes the last system for blastocyst culture after one or more medium changes (wherein, for example, the culture medium change may be performed on D2-D3 or D3-D4, for example on D3).

In another preferred embodiment, " day 5 to day 6 (D5-D6) " of *in vitro* culture includes D5±12 hours or D6±12 hours, preferably D5±6 hours, and more preferably D5 to D5+6 hours.

In another preferred embodiment, the said day of the in vitro culture is D5, that is, the fifth day after the culture starts, wherein the first day of the culture is set to D1.

In another preferred embodiment, in step (a), the *in vitro* culture of the blastocyst is *de novo* culture (that is, culture starts from D1 and does not undergo the change of the medium).

In another preferred embodiment, in step (a), the *in vitro* culture of the blastocyst is a staged culture.

In another preferred embodiment, in step (a), the *in vitro* culture of the blastocyst includes a first-stage blastocyst culture and a second-stage blastocyst culture.

In another preferred embodiment, the first-stage blastocyst culture includes culturing in a cleavage-stage culture medium on Day 1 (D1) to Day 3(D3), preferably, D1 to D3 ± 12h, more preferably, D1 to D3 ± 8h, more preferably, D1 to D3 ± 6h.

In another preferred embodiment, the second-stage blastocyst culture includes culturing in a blastocyst culture medium on Day 3 to Day 5.

In another preferred embodiment, the T1 time is 2 to 8 hours, such as 3 to 6 hours, preferably 3 to 5 hours.

In another preferred embodiment, the zona pellucida of the blastocyst is perforated in the second blastocyst culture system.

In another preferred embodiment, the opening size formed by zona pellucida perforation is 10-40 µm, preferably, 10-30 µm, more preferably, 10-20 µm.

In another preferred embodiment, the method has one or more of the following characteristics:
(i) a high signal-to-noise ratio, wherein the signal-to-noise ratio S₁/S₀>2, preferably, >5, more preferably, >10, where S₁ is the signal from the embryo and S₀ is the signal from the background;
(ii) low false-negative rate, wherein the false-negative rate is <8%, preferably, <5%, more preferably, <3%;
(iii) high accuracy, wherein the accuracy rate is ≥80%, preferably, 85%≥, more preferably, ≥90%.

In another preferred embodiment, the first blastocyst culture system is a culture system for monospermic fertilization (or an ICSI culture system).

In another preferred embodiment, the first blastocyst culture system is a culture system for polyspermic fertilization (or an IVF culture system).

In another preferred embodiment, in step (a), the blastocyst cultured *in vitro* is a monosperm-fertilized blastocyst (or an ICSI blastocyst).

In another preferred embodiment, in step (a), the blastocyst cultured *in vitro* is a polyspermy-fertilized blastocyst (or an IVF blastocyst).

In another preferred embodiment, the second blastocyst culture system contains only one blastocyst (embryo).

In another preferred embodiment, the second blastocyst culture system is a single embryo culture system comprising 10-60 microliters, preferably 10-50 microliters, or 10-30 microliters, more preferably, 10-15 microliters of culture medium.

In another preferred embodiment, in the step (c), the volume of the collected culture medium is 50-100% of the volume of the culture medium in the single embryo culture system, preferably, 70-100%, more preferably , 80-100%, optimally, 90-100%.

In another preferred embodiment, the step (d) further includes step (e):
(i) mixing the culture medium with a lysis solution to obtain a first mixture containing the culture medium and the lysis solution;
(ii) mixing the first mixture with a lytic enzyme, incubating, and then inactivating the lytic enzyme, thereby obtaining a lysate; and
(iii) performing genomic analysis on the lysate to identify the health status of the blastocyst (embryo).

In another preferred embodiment, the health status of the embryo includes: chromosome aneuploidy, mitochondrial copy number, whether the DNA content is normal, and pathogenic gene detection.

In another preferred embodiment, the genome analysis method includes NICS-INST amplification and analysis method. The amplification method of NICS-INST can refer to the instructions of the Universal Library Preparation Kit (trade name NICS-Inst™) from Xukang Medical Technology (Suzhou) Co., Ltd. Other genome analysis methods known in the art, including amplification methods and analysis methods, are also applicable to the present invention. For example, CN103890191 B discloses the use of MALBAC genome amplification method and the like.

In another preferred embodiment, the genome analysis method is selected from the group consisting of: second-generation sequencing, nucleic acid chip, immunofluorescence detection, fluorescent PCR detection, first-generation sequencing, third-generation sequencing, mass spectrometry, or a combination thereof.

In another preferred embodiment, the lysis solution contains components selected from the group consisting of: Tris buffer, chelating agent, hydrochloride salt, non-ionic surfactant, or a combination thereof.

In another preferred embodiment, the Tris buffer includes Tris-CI.

In another preferred embodiment, the concentration of the Tris buffer is 10-60 mM, preferably, 15-50 mM, more preferably, 20-45 mM, such as 30 mM.

In another preferred embodiment, the pH of the Tris buffer is 5-10, preferably 6-9, more preferably 7-8.

In another preferred embodiment, the chelating agent includes EDTA.

In another preferred embodiment, the concentration of the chelating agent is 0.2-8mM, preferably 0.3-6mM, more preferably 0.5-4mM, for example 2mM.

In another preferred embodiment, the hydrochloride salt is selected from the group consisting of KCl, NaCl, or a combination thereof.

In another preferred embodiment, the concentration of the hydrochloride salt is 5-60 mM, preferably, 8-40 nM, more preferably, 10-40 mM, such as 20 mM.

In another preferred embodiment, the non-ionic surfactant is selected from the group consisting of Triton X-100, Triton X-114, Tween 20, NP40, SDS, or a combination thereof.

In another preferred embodiment, the concentration of the non-ionic surfactant is 0.02-10%, preferably, 0.05-5%, more preferably, 0.1-3%, for example, 0.2%, based on the total weight of the lysis solution.

In another preferred example, in the step (e), in step (i), the volume ratio of the culture medium to the lysis solution is 1:10-10:1, preferably, 1:5-5:1, more preferably Land, 1:2-2:1.

In another preferred embodiment, the lytic enzyme is selected from the group consisting of proteinase K, Qiagen Protease, pepsin, papain, trypsin, lysozyme, or a combination thereof.

In another preferred embodiment, the concentration of the lytic enzyme is 1-25 µg/ml, preferably, 5-20 µg/ml, more preferably, 10-15 µg/ml.

In another preferred embodiment, the amount of the added lytic enzyme is 0.1-10 µl, preferably 0.5-6 µl, more preferably 0.8-3 µl.

In another preferred embodiment, the step (e)(ii) includes one or more features selected from the group consisting of:
(i) the incubation temperature is 20-70°C, preferably, 30-60°C;
(ii) the incubation time is 1 minute to 12 hours, preferably, 10min-6h, more preferably, 30min-2h;
(iii) the deactivation temperature is 60-100°C, preferably 75-95°C;
(iv) the inactivation time is 0.5-20 min, preferably 0.8-15 min.

In another preferred embodiment, in step (e) (iii), PCR is used for genome analysis. Preferably, the PCR reaction tube contains an amplification mixture, 0.5%-20% agent against PCR inhibitor, 5-20mM dNTP, 5-100µM NG and NT primers, 50-200µM amplification primers, 0.5-10 units of a nucleic acid polymerase, preferably, the agent against PCR inhibitor is selected from one or more of DMSO, betaine, formamide, glycerol and albumin; the nucleic acid polymerase is selected from one or more of Phi29 DNA polymerase, Bst DNA polymerase, Vent polymerase, Deep Vent polymerase, Klenow Fragment DNA polymerase I, MMLV reverse transcriptase, AMV reverse transcriptase, HIV reverse transcriptase, ultra-fidelity DNA polymerase, Taq polymerase, E.coli DNA polymerase, LongAmp Taq DNA polymerase and OneTaq DNA polymerase.

In another preferred embodiment, the amplification mixture comprises 10-25mM Tris-HCI, 5-25mM(NH4)2SO4, 5-30mM KCI, 0.5-5mM MgSO4, 0.1%-20% DMSO and 0.05- 5% Triton X-100.

In another preferred embodiment, the amplification mixture comprises, preferably, 15mM Tris-HCI, 15mM (NH4)2SO4, 20mM KCI, 1mM MgSO4, 5% DMSO and 2% Triton X-100.

In another preferred embodiment, the NG and NT primers contain a universal sequence and a variable sequence from the 5'-end to the 3'-end, wherein the universal sequence is composed of two or three of the four bases of G, A, C and T, provided that the universal sequence does not include G and C at the same time; the amplification primer includes the universal sequence and does not include the variable sequence.

In another preferred embodiment, the variable sequence is selected from the group consisting of: (N)ₙGGG, (N)ₙTTT, (N)ₘTNTNG, (N)ₓGTGG(N)_{y}, where N is any nucleotide capable of base pairing with natural nucleic acid, n is a positive integer selected from 3-17, m is a positive integer selected from 3-15, and x and y are each positive integer selected from 3-13.

In another preferred embodiment, in step (e) (iii), in the genome analysis by PCR, the thermal cycling procedure for whole genome amplification is as follows:
(1) at a first denaturation temperature between 90-98°C for 5-20s;
(2) at a first annealing temperature between 5-15°C for 5-60s, and at a second annealing temperature between 15-25°C for 5-60s, at a third annealing temperature between 25-35°C for 30-80s, at a fourth annealing temperature between 35-45°C for 5-60s, and at a fifth annealing temperature between 45-55°C for 5-60s;
(3) at a first extension temperature between 55-80°C for 10-150 min;
(4) at a second denaturation temperature between 90-98°C for 5-30s;
(5) at a sixth annealing temperature between 45-70°C for 10-30s;
(6) at a second extension temperature between 60-80°C for 1-10min;
(7) repeat steps (4) to (6) for 5 to 50 cycles;
(8) continue the extension reaction for 1-10min at a temperature between 60-80°C;
(9) store the amplified product in a refrigerator at 0-5°C.

In another preferred embodiment, in steps (e) (iii), the thermal cycling procedure for whole genome amplification during genome analysis by PCR is as follows:
(1) at a first denaturation temperature 95°C for 10s;
(2) at a first annealing temperature of 10°C for 45s, at a second annealing temperature of 20°C for 45s, at a third annealing temperature of 30°C for 60s, and at a fourth annealing temperature of 40°C for 45s, and at a fifth annealing temperature of 50°C for 45s;
(3) at a first extension temperature of 62°C for 90 minutes;
(4) at a second denaturation temperature of 95°C for 20s;
(5) at a sixth annealing temperature of 59°C for 20s;
(6) at a second extension temperature of 72°C for 3min;
(7) repeat steps (4) to (6) for 10 to 30 cycles;
(8) continue the extension reaction at 72°C for 5 minutes;
(9) store the amplified product in a refrigerator at 4°C.

In another preferred embodiment, the method is non-therapeutic and non-diagnostic.

The second aspect of the present invention provides a method for preparing a gene test sample or a chromosome test sample, including the steps:
(a) providing a first blastocyst culture system, which comprises an *in vitro* cultured blastocyst on Day 5 (D5) to Day 6 (D6);
(b) transferring the blastocysts into a second blastocyst culture system containing a fresh blastocyst culture medium, and culturing in the exchanged medium for a time of T1, thereby obtaining a spent blastocyst culture medium;
(c) collecting the spent blastocyst culture medium, thereby obtaining a cell-free spent blastocyst culture medium, i.e., the test sample.

In another preferred embodiment, the second blastocyst culture system contains only one blastocyst (embryo).

In another preferred embodiment, the second blastocyst culture system is a single embryo culture system, containing 10-60 microliters, preferably 10-50 microliters, more preferably 10-15 microliters of culture medium.

In another preferred embodiment, in step (c), the volume of the test sample is 50-100% of the volume of the culture medium in the second blastocyst culture system, preferably 70-100%, more preferably, 80-100%, optimally, 90-100%.

The third aspect of the present invention provides a kit for detecting the health status of blastocysts using blastocyst culture medium, which includes:
(i) a first container, and a cleavage-stage culture medium contained in the first container;
(ii) a second container, and a first blastocyst culture medium contained in the second container;
(iii) a third container, and a second blastocyst culture medium contained in the third container; and
(iv) labels or instructions.

In another preferred embodiment, the first container, the second container, and the third container are different.

In another preferred embodiment, the cleavage-stage culture medium in the first container is used for cleavage-stage embryo culture on D1 to D3, preferably, to D3±12h, more preferably, to D3±8h, more preferably, to D3±6h.

In another preferred embodiment, the first blastocyst culture medium in the second container is used for blastocyst culture on D3 to D5.

In another preferred embodiment, the second blastocyst culture medium in the third container is used for blastocyst culture at the time after D5 to D6.

In another preferred embodiment, the first blastocyst culture medium and the second blastocyst culture medium have the same composition, and both are G-2 PLUS medium (Vitrolife) or Quinn's Advantage Protein Plus blastocyst medium (SAGE).

In another preferred embodiment, the cleavage-stage culture medium is G-1 PLUS medium (Vitrolife) or Quinn's Embryo maintenance medium (SAGE).

The fourth aspect of the present invention provides a device for assisting in diagnosing the health status of blastocysts, including:
(a) a culture module, which includes a culture module for a later-stage blastocyst, functioning to exchange the medium of the cultured blastocyst on Day 5 to Day 6, and to perform the later-stage culture of the blastocyst for a period of time (T1);
(b) a sampling module, functioning to collect from the later-stage blastocyst culture module, the spent culture medium of the blastocyst as a test sample;
(c) a detection module, functioning to perform a gene detection on the test sample collected from the sampling module, thereby obtaining a detection result;
(d) an evaluation module, functioning to determine based on the detection result the health status of the blastocyst, thereby providing the evaluation result of the blastocyst health status;
(e) an output module, functioning to output the evaluation result of the health status of the blastocyst.

In another preferred embodiment, the T1 time is 2-8h, such as 3-6h, preferably 3-5h.

It should be understood that within the scope of the present invention, the above-mentioned technical features of the present invention and the technical features specifically described in the following (such as the embodiments) can be combined with each other to form new or preferred technical solutions, which will not described one by one here due to the length limitation of the text.

### Description of the drawings

Figure 1 shows a schematic diagram of the difference between the conventional sampling method and the sampling method of the present invention in the exposure time of a test sample.
Figure 2 shows the CNV analysis results of the culture medium collected using the conventional D3-D5 sampling method.
Figure 3 shows the CNV analysis results of the culture medium collected using the D5 sampling method of the present invention.

### Detailed description of the invention

After a long-term, extensive and in-depth research, through a large number of screenings and tests, the inventors unexpectedly discovered for the first time that: by using the 10-20 microliter blastocyst culture system of the present invention to culture an embryo (blastocyst) in a blastocyst culture medium to Day, D5 to D6, and then transferring the embryo to a fresh blastocyst culture medium for culture, and collecting a small amount of culture medium from the fresh blastocyst culture medium for testing, the embryo health status (such as chromosome aneuploidy, mitochondrial copy number, DNA content is normal, and pathogenic gene detection) can be accurately detected with extremely low false-negative rate (<8%, preferably, <5%) . The method can greatly eliminate the risk of interference by contaminants from extra sperm and maternal-derived granular cells. On this basis, the inventor completed the present invention.

As used herein, the terms "blastocyst" and "embryo" are used interchangeably and refer to embryo at the final stage of *in vitro* culture, which is usually formed on the 5^{th} to the 7^{th} day after fertilization of the egg.

As used herein, a depleted medium refers to a spent medium, for example, a medium separated from a culture system which has been used to culture embryos.

As used herein, a de novo culture refers to an embryo culture process in which the culture medium is not exchanged from the start of the culture process to the end of the culture process. For example, a de novo culture on D1 to D5 means that the embryo culture process starts from D1 and continues to D5, during which the culture medium is not replaced.

As used herein, a staged culture refers to an embryo culture process in which the embryo undergoes one or more medium exchanges. For example, a staged culture on D1 to D5 means that the embryo culture process starts from D1 and continues to D5, during which the embryo undergoes one or more medium exchanges, for example, on D1 to D3, embryos are cultured in a cleavage-stage medium; and on D3 to D5, embryos are cultured in a blastocyst culture medium.

As used herein, "Dx" (where x = a positive integer of 1, 2, 3....) means that, with the start of the in vitro culture of a fertilized oocyte set to day 1 (D1), Dx is the X day of the in vitro culture.

As used herein, "D3-D5 sampling" means that a culture medium is used to culture embryos from D3 to D5, and a sample is collected from the spent culture medium (i.e., the depleted culture medium) on D5. Therefore, the culture medium sample obtained by this sampling method will be in contact with the cultured embryo for a period of time (i.e., the exposure time of the test medium sample), which is at least about 48 hours or more.

As used herein, "on-the-day sampling" refers to collecting a sample from a spent culture medium (i.e., depleted culture medium) on the day of culture medium exchange and after embryo is cultured in the exchanged medium for a short period. Therefore, the time for the culture medium sample obtained by this sampling method to contact the cultured embryo (i.e., the exposure time of the test sample) will be short. In the present invention, it is preferable that the exposure time does not exceed 8 hours, such as 3-6 hours, more preferably 3-5 hours.

As used herein, "lysis solution" refers to a lysis buffer used to decompose proteins and cells in a culture medium sample. In some embodiments, the lysis solution may or may not include a lytic enzyme. In some embodiments, the lysis solution and the lytic enzyme can be added to the culture medium sample at the same time. In other embodiments, the lytic enzyme is supplemented after mixing the lysis solution with the culture medium sample.

As used herein, " *in vitro* incubation time of test sample ", "culture time of test sample " and " exposure time of test sample " are used interchangeably, which means the length of time for which the culture medium used for sampling has been *in vitro* in contact with embryos, or has been incubated with embryo.

As used herein, "CNV" refers to chromosome copy number variants. CNV nomenclature complies with the International System for Human Cytogenetic Nomenclature (ISCN). See, Shaffer LG, Slovak ML, Campbell LJ (2009): ISCN 2009 an international system for human cytogenetic nomenclature, Human Genetics volume 126, Article number: 603 (2009).

### IVF and IVF embryos

IVF refers to in vitro fertilization, specifically refers to in vitro fertilization combined embryo transfer technology, also known as test tube baby, by which an oocyte and a sperm are taken, placed in a test tube to fertilize, and then the fertilized oocyte (i.e., precursor of embryo) is transplanted back into the mother's uterus to develop into a fetus.

"Polysperm-fertilized blastocyst" or "IVF blastocysts" refer to a blastocyst obtained by *in vitro* co-culturing more than one sperm with an oocyte to fertilize using IVF technology.

### ICSI and ICSI embryos

ICSI (Intracytoplasmic sperm injection) is a technology by which a single sperm is microinjected into the cytoplasm of an egg, which is the second generation of "test tube baby". This technology uses a microscopic operating system to inject a single sperm into an egg to fertilize it. "monosperm-fertilized blastocyst" or "ICSI blastocyst" refers to a blastocyst obtained by using ICSI, the technique of intracytoplasmic sperm microinjection.

### Detection method

The present invention provides a detection method that performs a genetic detection on a depleted medium used for a blastocyst culture (i.e., the culture medium separated from a culture system which has been used to culture a blastocyst for a period of time), thereby identifying the health status of the embryo.

In the present invention, the method for genetic detection on a depleted culture medium used for blastocyst culture is not particularly limited, and conventional methods can be used for detection, such as second-generation sequencing, nucleic acid chip, immunofluorescence detection, fluorescent PCR detection, first-generation sequencing, third-generation sequencing, mass spectrometry detection, or a combination thereof.

In one embodiment, the detection method includes the following steps:
(a) providing a first blastocyst culture system, which comprises a blastocyst on Day 5 (D5) to Day 6 (D6) of *in vitro* culture;
(b) transferring the blastocyst into a second blastocyst culture system containing a fresh blastocyst culture medium, and culturing in the exchanged medium for a time of T1, thereby obtaining a spent blastocyst culture medium;
(c) collecting the spent blastocyst culture medium, thereby obtaining a cell-free spent blastocyst culture medium, which is the test sample; and
(d) performing a genetic assay to identify the health status of the blastocyst.

In a preferred embodiment, the step (d) further includes step (e):
(i) mixing the culture medium with a lysis solution to obtain a first mixture containing the culture medium and the lysis solution;
(ii) mixing the first mixture with a lytic enzyme, incubating, and then inactivating the lytic enzyme, thereby obtaining a lysate; and
(iii) Perform genomic analysis on the lysate to identify the health status of the blastocyst (embryo).

In a specific embodiment, the method according to the present invention by using blastocyst culture medium as a test sample to reflect the health status of embryos include the following main steps:
(A) an oocyte is fertilized by IVF or ICSI, and after fertilization, the fertilized oocyte is transferred to a culture medium and cultured to the D3 cleavage stage, and then the embryo is transferred to a blastocyst culture medium on D3 and cultured to D5-D6;
(b) blastocyst grading is performed, and the well-developed blastocyst is transferred to another fresh blastocyst culture medium on D5±0.5 days, and a laser puncture instrument is used to perforate the zona pellucida under a high-power microscope to make the embryo shrink, the blastocyst is removed out 3-5 hours later and subjected to vitrification, and the remaining spent blastocyst culture medium is the test sample to be collected;
(c) the spent blastocyst culture medium obtained in step (b) is transferred into a lysis solution, and after centrifugation, the sample is subjected to a whole genome amplification, including but not limited to, the amplification of NICS-INST, and an analysis, thereby obtaining the test result reflecting the embryo's health status.

The present invention performs a culture medium exchange on D5±0.5 days and a 3-5 hours short-time culture before the blastocyst culture medium is collected as a test sample. By this, the accuracy of the test result is significantly improved, the false negative rate is reduced to less than 5%, and the interference from external substances, especially maternal-derived interference, is avoided to a great extent. In addition, by this, the method according to the present invention is applicable to both ICSI and IVF fertilization.

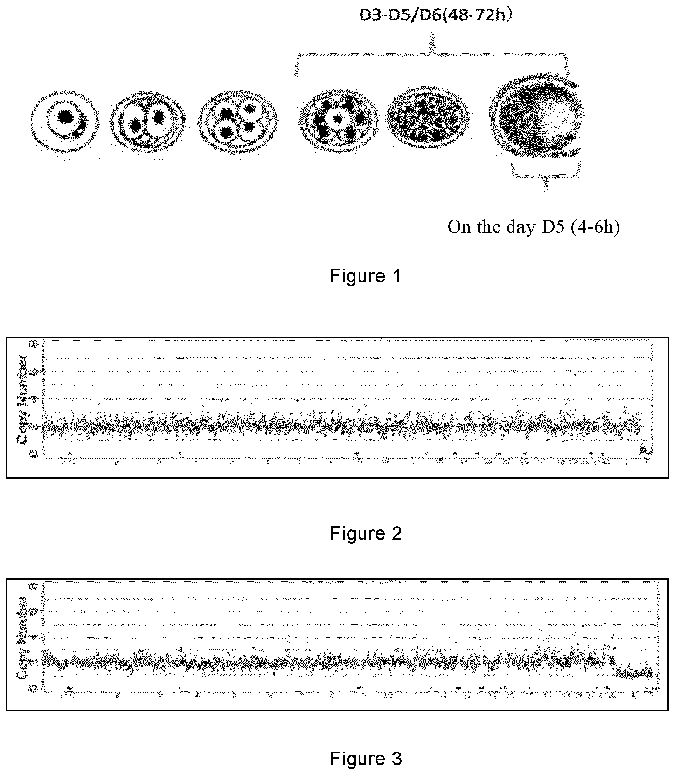

In the present invention, it is believed that the following contributes to the improved accuracy of the detection result: First, compared with the exposure time of 2-3 days in the conventional operation method (as shown in Figure 1), the exposure time of the test sample according to the present invention is shorten to a very short period, by which the interference from external substances, especially the maternal-derived interference, is avoided to the maximum extent possible, and irrespective of the fertilization process, the accuracy of the detection results is greatly improved. Second, embryo has the ability to repair itself, and abnormal fragments are released into the culture medium, which may affect the accuracy in the conventional culture method. In the present invention, sampling is made at the time of 3-5 hours incubation after D5 culture medium exchange, so that the culture time for embryo in the exchanged medium is short. The self-repairing of embryo occurs during the D3-D5 culture period and before the D5 culture medium exchange, with most of abnormal fragments released during the D3-D5 culture period. This causes a low release rate of abnormal fragments in the test sample collected according to the present invention, and consequently an improved accuracy of the detection result. Before the present invention is filed, generally the culture medium of ICSI fertilized embryo is collected, but no one has tried to collect the culture medium of IVF embryo. The most important factor for external interference is the excessively long *in vitro* incubation time of the test sample. The present invention adopts a short-time incubation method for collecting culture medium sample. The method avoids external interference from such as sperm and granular cells due to excessively long incubation time, which will reduce the ratio of embryo-derived DNA in the test sample and affect the accuracy of the results.

The amount of the test sample in the step (c) is 8-15ul, preferably 10ul. The amount of the fresh blastocyst culture medium (i.e., the second blastocyst culture medium) is preferably 10-15ul.

Preferably, the blastocyst grading includes selecting a well-developed embryo that has developed to stage 4 or higher.

The perforation in step (b) increases the release of blastocoel fluid, that is, the release of free nucleic acid, so that the initial amount of the nucleic acid substance is increased in the test sample under the premise of short-term incubation for 3-5 hours, to a level sufficient for subsequent amplification and detection, thereby ensuring the success detection rate can reach more than 97%. Embryos are usually observed, graded and frozen in the blastocyst stage. It is believed that operations at the blastocyst stage will not cause damage to the embryo. The present invention also chooses the D5 blastocyst stage to perform laser drilling and *in vitro* incubation, so those operations are performed without affecting embryonic development.

In the present invention, step (b) may also include a step of washing embryos before changing the culture medium on D5, which is helpful to further improve the accuracy of the detection results.

In the present invention, in the step (a), after the exchange of a blastocyst culture medium on D3, multiple embryos can be mixed in culture, or a single embryo culture can be performed.

### Sample preparation and testing

The present invention also provides a method for preparing a genetic test sample or a chromosome test sample, including the steps:
(a) providing a first blastocyst culture system, which comprises a blastocyst on Day 5 (D5) to Day 6 (D6) of *in vitro* culture;
(b) transferring the blastocysts into a second blastocyst culture system containing a fresh blastocyst culture medium, and culturing in the exchanged medium for a time of T1, thereby obtaining a spent blastocyst culture medium;
(c) collecting the spent blastocyst culture medium, thereby obtaining a cell-free spent blastocyst culture medium, i.e., a test sample.

In another preferred embodiment, the test sample obtained by the method can be used for genetic testing to identify the health status of the embryo.

### Zona pellucida perforation

An embryo grows to the blastocyst stage, and the blastocyst is positioned on the operating table. A site far from the inner cell mass and with thin trophectoderm is chosen, a laser beam is used to punch the blastocyst cavity, and create a small hole in the zona pellucida to allow the Blastocoel fluid release into the blastocyst culture medium. The opening size of the zona pellucida is not particularly limited. A preferred opening size of the zona pellucida is 10-40 µm, preferably, 10-30 µm, and more preferably, 10-20 µm.

In the present invention, the perforation of the zona pellucida increases the release of blastocoel fluid, that is, the release of free nucleic acid, which leads to an increase in the initial amount of the nucleic acid substance in the test sample under the premise of a short incubation of 3-5 hours. As a consequence, the test sample has enough nucleic acid substance for subsequent amplification and detection, thereby ensuring the success detection rate can reach more than 97%.

If the laser drilling operation is not performed, the detection success rate is also high, up to about 70%.

### Detection kit

In the present invention, a kit for detecting the health status of embryos using blastocyst culture medium is provided, which includes:
(i) a first container, and a cleavage-stage culture medium contained in the first container;
(ii) a second container, and a first blastocyst culture medium contained in the second container;
(iii) a third container, and a second blastocyst culture medium contained in the third container; and
(iv) labels or instructions.

### Device for assisting in the diagnosis of embryo health

In the present invention, a device for assisting in diagnosing the health status of embryos is provided, including:
(a) a culture module, which includes a culture module for a later-stage blastocyst, functioning to exchange the medium of the cultured blastocyst on Day 5 to Day 6, and to perform the later-stage culture of the blastocyst for a period of time (T1);
(b) a sampling module, functioning to collect from the later-stage blastocyst culture module, the spent culture medium of the blastocyst as a test sample;
(c) a detection module, functioning to perform a gene detection on the test sample collected from the sampling module, thereby obtaining a detection result;
(d) an evaluation module, functioning to determine based on the detection result the health status of the blastocyst, thereby providing the evaluation result of the blastocyst health status;
(e) an output module, functioning to output the evaluation result of the health status of the blastocyst.

The present invention also relates to the use of said module (a), optionally in combination with modules (b)-(e), in the preparation of a device used in the method for evaluating the health status of blastocysts according to the present invention.

### The main advantages of the present invention include:

(1) In the present invention, it was discovered for the first time that by transferring a blastocyst on D5-D6 of *in vitro* culture to a fresh blastocyst culture medium for a short-term culture and then separating the spent culture medium therefrom and taking a small amount of the culture medium for genetic testing, the embryo's health status (such as whether chromosomes are abnormal, chromosome aneuploidy, mitochondrial copy number, whether the DNA content is normal, pathogenic gene testing, etc.) can be detected in an extremely high accuracy and with a very low false-negative rate (less than 8%, preferably, less than 5%), and the risk of the interference by contaminants from extra sperm and maternal-derived granular cells can be greatly reduced.
(2) The present invention is applicable to a single embryo culture system, that is, only one embryo is cultured in one culture medium, and the detection result of the embryo's health status obtained by the system is more accurate.
(3) The method according to the present invention is a universal method, which can be used for monosperm-fertilized embryos, and can also be used for polysperm-fertilized embryos, and polysperm-fertilized embryos are preferred.
(4) The method of the present invention may include a step of zona pellucida drilling, by which the detection result can be further improved.
(5) The method of the present invention has a high signal-to-noise ratio.
(6) In the method of the present invention, the collection operation of embryo culture medium is performed after the embryo has been cultured to Day 5 (D5). The operation time is extremely short, and the incubation time of the test sample is controllable, and the risk of external interference is low. In addition, the sample collection method is not limited by fertilization process, and is a culture medium sampling method applicable to both IVF and ICSI embryos.
(7) The process according to the present invention using D5 medium exchange and a short-term culture has the advantage of controlling the *in vitro* incubation time of the test sample, thereby controlling external interferences such as maternal-derived interference and sperm-derived interference to the greatest extent, so that the application of the present invention is not limited to ICSI embryos, but also can be applied to IVF embryos.

The present invention will be further explained below in conjunction with specific examples. It should be understood that these examples are only used to illustrate the present invention and not to limit the scope of the present invention. The experimental methods that do not indicate specific conditions in the examples usually follow the conventional conditions or the conditions recommended by the manufacturer. Unless otherwise stated, percentages and parts are weight percentages and parts by weight.

Unless otherwise specified, the materials used in the present invention are all commercially available products.

### General method

Obtaining blastocyst culture medium:
(1) *in vitro* culture fertilized oocytes (obtained from a volunteer couple, wherein oocytes are obtained from female volunteer, and sperms are from male volunteer, and IVF or ICSI is used for *in vitro* fertilization to obtain fertilized oocytes) to the third day (D3), and further cultur, with or without medium exchange, to the 5th day (D5)/the 6th day (D6);
(2) When the embryos develop to blastocysts above stage 4, transfer the embryos to at least three droplets of 30ul blastocyst culture medium (ensuring that there was only one embryo in one droplet), and with gently blowing each droplet, rinse the droplets 2-3 times, then transfer embryos into 10-15ul microdroplets that have been equilibrated overnight at 37°C, and 5% CO2 and 5% O2, and use a laser drilling instrument to perforate the zona pellucida under a high-power microscope to make the embryos shrink; continue culturing for 3-5 hours or 3-6 hours.
(3) transfer the culture medium of the short-term cultured blastocyst (about 10-15 microliters) obtained in step (2) to 5 microliters of lysis solution (30 mM Tris-CI, pH7.8, 2 mM EDTA, 20 mM KCI, 0.2% Triton X-100), mark the sample name on the collection tube, and centrifuge for 30 seconds in a microcentrifuge. The sample can immediately be used for the whole genome amplification or stored at -20°C or -80°C.

The detection method of the present invention is carried out with reference to the instructions of the Universal Library Preparation Kit (trade name NICS-Inst™, Xukang Medical Technology (Suzhou) Co., Ltd.)

### Example 1

This embodiment relates to a comparison between on-the-day D5 sampling method of the present invention and the conventional D3-D5 sampling method.

In brief, fertilized oocytes were cultured *in vitro* to D3, and then the resulted embryos were transferred to a blastocyst culture medium, and were continually cultured to D5. According to the conventional D3-D5 sampling method, the blastocyst culture mediums on D5 were taken as the samples of the "D3-D5 sampling" method. After that, according to the method of the present invention, the D5 blastocysts were transferred to a fresh blastocyst culture medium, and "on-the-day sampling" was performed on the day of D5 according to the general method described above. The CV test results of "on-the-day D5 sampling" were compared with the test results of "D3-D5 sampling" from the same embryos, and compared with the test results of the whole embryos. The results from whole embryos were used as the gold standard. When a test result from a whole embryo presents abnormal chromosome copy number and the test result from culture medium presents normal chromosome copy number, the culture medium test result is judged as false negative. The false-negative rate is the proportion of false negative test results in all test results.

As shown by a large amount of experimental data, the accuracy of the detection result of the present invention is significantly improved, and the false negative rate is reduced to less than 5%. The false negative rate of the conventional D3-D5 sampling (130 sets of samples) and the false negative rate of the "on-the-day D5 sampling" of the present invention (62 sets of samples) are shown below:

| | D3-D5 sampling | On-the-Day D5 sampling |
|---|---|---|
| False negative rate | 12.3% | 1.3% |
| Accuracy rate | 73.1% | 91% |

As shown in the above table, by blastocyst culture medium exchange on D5 with a short-term culture before sampling, the false negative rate can be controlled at 1.6%. In contrast, by the D3-D5 sampling method, the maternal-derived interference cannot be effectively controlled, and the false negative rate is 12.3%, prone to cause clinical misdiagnosis.

Some of the false negative cases caused by D3-D5 are as follows: (the table shows the results of CNV chromosome karyotype analysis using different culture media)

| Test results of D3-D5 sampling | Test results of on-the-day D5 sampling |
|---|---|
| 46,XX | 46,XN,+9(X3,mos,70%) |
| 46,XX | 45,XN,,-22(×1) |
| 46,XX | 46,XN,-3(×1),+13(×3) |
| 46,XX | 45,XN,-7(×1) |
| 46,XX | 46,XN"+8(×3,mos*),+9(×3),-13(×1,mos*) |
| 46,XX | 45,XN,-6(×1,mos*),-21(×1) |
| 46,XX | 47,XN,+22(×3) |

In the present invention, by blastocyst culture medium exchange on D5 with a short-term culture before sampling, the maternal-derived interference can reduced to the greatest extent, as shown in Fig. 2 and Fig. 3. Figures 2 and 3 show the chromosome copy number (CN) profiles of a male embryo, respectively obtained by the method of the present invention and by the conventional D3-D5 sampling method. The horizontal axis on the CN profiles is the chromosomes, which are arranged in the order of autosomes 1-22 and sex chromosomes X and Y; the ordinate axis is the copy number. For normal human embryos (diploid), the autosomal copy number should be 2. Sex chromosomes are two copies of X (female), or one copy each of X and Y (male).

In the D3-D5 sampling method, it is difficult to avoid the phenomenon of maternal-derived interference when collecting culture medium, which may cause inaccurate determination of CNV (Copy number variants). For example, the result of 46XY (male) is interpreted as 46XY/XX (male and female Mosaic), as shown in Figure 2. In the D5 on-the-day sampling method of the present invention, the culture medium is collected on the day of D5, which can minimize maternal interference and restore the true CNV results, as shown in Figure 3, where the CNV is interpreted as 46XY (male).

By comparative experiments, it was also found that when the blastocyst culture medium collected by the present invention is used for gender detection, whether it is an ICSI or IVF embryo has no effect on the accuracy of gender determination compared with the detection by using whole embryos, and there is no paternal interference or maternal interference to cause false negatives. Some cases are as follows:

| Test results of on-the-day D5 sampling | Test results of corresponding whole-embryo | Fertilization process |
|---|---|---|
| 46,XX | 46,XX | ICSI |
| 47,XX,+8(×3) | 47,XX,+8(×3) | ICSI |
| 46,XY | 46,XY | ICSI |
| 47,XY,+15(×3) | 46,XY | ICSI |
| 46,XY | 46,XY | ICSI |
| 46,XX,+20(×3),-22(×1) | 45,XX,-22(×1) | ICSI |
| 47,XY,+7(×3) | 46,XY | ICSI |
| 46,XX | 46,XX | ICSI |
| 48,XY,+5(×3),+14(×3) | 47,XY,+14(×3) | IVF |
| 47,XX,+13(×3) | 47,XX,+13(×3) | IVF |
| 46,XX | 46,XX | IVF |
| 46,XY | 46,XY | IVF |
| 47,XY,+22(×3) | **47,XY,+22**(×3) | IVF |
| 45,XY,-7(×1) | 46,XY | IVF |
| 46,XX | 46,XX | IVF |
| 46,XY | 46,XY | IVF |

Therefore, the method of the present invention has very good accuracy and a very low false negative rate.

### Comparative Example 1

The method is the same as in Example 1, the differences are that culture is continued to day 3 (D3) to day4 (D4), and the culture medium is changed on D4, and after 24 or 48 hours of continuous culture, the spent blastocyst culture medium is taken for detection. The test results conducted on 19 sets of samples showed that the accuracy of the test results of embryo health (such as embryo chromosomal abnormality) was 84.2% (16/19), and the false negative rate was 10.5% (2/19). After increasing the sample size, using the method of Comparative Example 1, the results show that the accuracy of this method became smaller with higher false-negative rate.

### Discussion

D4 embryo usually develops into embryo of morula stage, which stage is the critical period for embryo to develop into blastocyst. The stage next to morula-stage is blastocyst stage. The blastocyst forming rate is a key indicator of embryo development. After a blastocyst forms, *In vitro* operations such as freezing and transplantation can be considered. Thus, clinically embryos at the morula-stage of D4 are usually not subjected to *in vitro* operations such as medium exchange, so as not to affect the blastocyst formation rate.

Embryos are observed, graded, and frozen in the blastocyst stage. The operation at the blastocyst stage will not cause damage to the embryos. In the present invention, the blastocyst stage of D5 is selected for laser drilling and in vitro incubation, and therefore, those operations are carried out under the premise of not affecting embryo development.

All documents mentioned in the present invention are cited as references in this application, as if each document was individually cited as a reference. In addition, it should be understood that after reading the above teaching content of the present invention, those skilled in the art can make various changes or modifications to the present invention, and these equivalent forms also fall within the scope defined by the appended claims of the present application.

## Claims

1. An in vitro method for detecting the health status of blastocysts using blastocyst culture medium, **characterized in that** it comprises the steps of:
(a) providing a first blastocyst culture system, which comprises a blastocyst on Day 5 (D5) to Day 6 (D6) of *in vitro* culture;
(b) transferring the blastocysts into a second blastocyst culture system containing a fresh blastocyst culture medium, and culturing in the exchanged medium for a time of T1, thereby obtaining a spent blastocyst culture medium;
(c) collecting the spent blastocyst culture medium, thereby obtaining a cell-free spent blastocyst culture medium, i.e., a test sample; and
(d) performing a genetic assay to identify the health status of the blastocyst.

2. The method according to claim 1, wherein in step (a), the *in vitro* culture of the blastocyst is a de novo culture or a staged culture.

3. The method of claim 1, wherein in step (a), the *in vitro* culture of the blastocyst includes a first-stage blastocyst culture and a second-stage blastocyst culture.

4. The method according to claim 3, wherein the first-stage blastocyst culture includes culturing on Day 1 to Day 3 in a cleavage-stage culture medium.

5. The method of claim 3, wherein the second-stage blastocyst culture includes culturing on Day 3 to Day 5 in a blastocyst culture medium.

6. The method of claim 1, wherein the T1 time is 2 to 8 hours, for example, 3 to 6 hours, preferably 3 to 5 hours.

7. The method according to claim 1, wherein the method has one or more of the following characteristics:
(i) a high signal-to-noise ratio, wherein the signal-to-noise ratio S₁/S₀>2, preferably, >5, more preferably, >10, where S₁ is the signal from the embryo and S₀ is the signal from the background;
(ii) low false-negative rate, wherein the false-negative rate is <8%, preferably, <5%, more preferably, <3%;
(iii) high accuracy, wherein the accuracy rate is ≥80%, preferably, 85%≥, more preferably, ≥90%.

8. A method for preparing a sample for a gene detection or for a chromosome detection, **characterized in that** it comprises the steps:
(a) providing a first blastocyst culture system, which comprises a blastocyst on Day 5 (D5) to Day 6 (D6) of *in vitro* culture;
(b) transferring the blastocysts into a second blastocyst culture system containing a fresh blastocyst culture medium, and culturing in the exchanged medium for a time of T1, thereby obtaining a spent blastocyst culture medium;
(c) collecting the spent blastocyst culture medium, thereby obtaining a cell-free spent blastocyst culture medium, i.e., a test sample.

9. A use of a culture module in the preparation of a product used in the method of claim 1, wherein the culture module comprises a culture module for a later stage blastocyst, functioning to exchange the medium of the cultured blastocyst on Day 5 to Day 6, and to perform the later-stage culture of the blastocyst for a period of time (T1).

10. A device for assisting in diagnosing the health status of a blastocyst, **characterized in that** it comprises:
(a) a culture module, which includes a culture module for a later stage blastocyst, functioning to exchange the medium of the cultured blastocyst on Day 5 to Day 6, and to perform the later-stage culture of the blastocyst for a period of time (T1);
(b) a sampling module, functioning to collect from the later-stage blastocyst culture module, the spent culture medium of the blastocyst as a test sample;
(c) a detection module, functioning to perform a gene detection on the test sample collected from the sampling module, thereby obtaining a detection result;
(d) an evaluation module, functioning to determine based on the detection result the health status of the blastocyst, thereby providing the evaluation result of the blastocyst health status;
(e) an output module, functioning to output the evaluation result of the health status of the blastocyst.

11. The method of claims 1-8, wherein the method further comprises, after exchanging the medium and before collecting the test sample, perforating the zona pellucida of the blastocyst.

12. The use of claim 9 and the device of claim 10, wherein the culture module further comprises a device for perforating the zona pellucida of the blastocyst after the medium is exchanged.
